Europäisches Patentamt

European Patent Office

Office européen des brevets

(11) Veröffentlichungsnummer: **0 157 394**
**B1**

(12) # EUROPÄISCHE PATENTSCHRIFT

(45) Veröffentlichungstag der Patentschrift:
24.05.89

(51) Int. Cl.⁴: **A 61 M 25/00**

(21) Anmeldenummer: **85103853.9**

(22) Anmeldetag: **30.03.85**

(54) **Venenkatheter mit Führungsmandrin zur EKG-Abnahme.**

(30) Priorität: **03.04.84 CH 1686/84**

(43) Veröffentlichungstag der Anmeldung:
**09.10.85 Patentblatt 85/41**

(45) Bekanntmachung des Hinweises auf die Patenterteilung:
**24.05.89 Patentblatt 89/21**

(84) Benannte Vertragsstaaten:
**AT BE CH DE FR GB IT LI LU NL SE**

(56) Entgegenhaltungen:
**EP-A- 0 082 504**
**DE-A- 2 306 519**
**DE-A- 2 736 623**
**DE-A- 3 120 012**
**DE-A- 3 417 479**

(73) Patentinhaber: **Sterimed Gesellschaft für medizinischen Bedarf mbH, Fasanerieweg 15-17 Postfach 215, D-6600 Saarbrücken 3 (DE)**

(72) Erfinder: **Kramann, Bernhard, Preysingstrasse 11, D-8000 München 51 (DE)**
Erfinder: **Rust, Meinhard, Knappertsbuschstrasse 12, D-8000 München 81 (DE)**

(74) Vertreter: **Suchy, Herbert, Dr., Byk Gulden Lomberg Chemische Fabrik GmbH Byk-Gulden-Strasse 2 Postfach 6500, D-7750 Konstanz (DE)**

## Beschreibung

Gebiet der Technik

Die Erfindung betrifft einen Venenkatheter mit einem Katheterschlauch und einem kunststoffummantelten, elektrisch leitfähigen Metalldraht als Führungsmandrin, über den zum Zwecke der Lagekontrolle ein EKG abgeleitet werden kann.

Stand der Technik

Katheterungen zentraler Venen (Hohlvene, Vena cava) spielen eine wichtige Rolle bei der Schockbehandlung, bei der Bestimmung des zentralvenösen Blutdrucks oder dessen Überwachung bei operativen Eingriffen sowie bei der parenteralen Ernährung.

In den meisten Fällen zentralvenöser Katheterungen ist es wünschenswert, daß das distale Ende des Katheters unmittelbar vor dem Vorhof des Herzen zu liegen kommt, wobei sicher ausgeschlossen sein muß, daß das distale Ende in den Vorhof des Herzens hineinragt. Zur Vermeidung der aufwendigen und den Patienten belastenden Lagekontrolle mittels Röntgenstrahlen unter Verwendung von Röntgenkontrastmitteln wurde vorgeschlagen, den Venenkatheter mit einer Elektrolytlösung zu füllen und über die elektrolytisch leitende Flüssigkeitssäule das EKG abzugreifen. Je nachdem, ob die Spitze des Katheters sich außerhalb oder innerhalb des Herzens befindet, beobachtet man deutlich unterschiedliche EKGs (M.H. Trujillo et al, American Heart Journal 84 (4), 451–455 (1972)).

In der DE-OS 31 20 012 wird zur Vermeidung der umständlichen und mit dem Risiko bakterieller Kontamination behafteten Manipulationen beim Einfüllen der Elektrolytlösung vorgeschlagen, die distale Spitze des Führungsmandrins elektrisch leitend auszubilden, eine elektrisch leitende verbindung zum proximalen Ende des Führungsdrahtes herzustellen und das EKG direkt über den Führungsdraht abzuleiten.

Nachteilig an diesem Vorschlag ist, daß die metallische Spitze des Führungsmandrins beim Vorschieben des Katheters in der Gefäßwand zu gefährlichen Perforationen führen kann. Hinzu kommt, daß es zu lebensbedrohenden Störungen (Herzflimmern) kommen kann, wenn die metallische Spitze des Führungsdrahtes mit der Herzwand in Berührung kommt und störende Potentiale, beispielsweise durch elektrostatische Aufladungen, auf die Herzwand übertragen werden.

Der vorliegenden Erfindung liegt die Aufgabe zugrunde, einen Venenkatheter der in Rede stehenden Gattung so zu gestalten, daß die Gefahr der Gefäßperforationen und des Erzeugens von Herzflimmern weiter verringert wird.

Im Zusammenhang mit dieser Erfindung soll der Begriff «distal» als «untersucherfern» und der Begriff «proximal» als «untersuchernah» verstanden werden.

Beschreibung der Erfindung

Die Lösung dieser Aufgabe wird dadurch erreicht, daß die Kunststoffummantelung im Bereich des distalen Endes des Metalldrahts eine oder mehrere Aussparungen aufweist, die sich innerhalb des Lumens des Katheterschlauches befinden. In einer vorteilhaften Ausführungsform befinden sich die Aussparungen des Kunststoffmantels in einer Entfernung von 1 bis 30 mm, vorzugsweise 1 bis 10 mm vom distalen Ende des Metalldrahts. In einer besonderen erfindungsgemäßen Ausführungsform ragt die Kunststoffummantelung in einer Länge von 1 bis 6, vorzugsweise 1,5 bis 2,5 cm über das distale Ende des Metalldrahts hinaus. In einer weiteren erfindungsgemäßen Ausführungsform ist das überragende Ende in seiner ganzen Länge oder teilweise als halbkreisförmig gekrümmtes distales Ende ausgeführt. Weiterhin kann am proximalen Ende des Führungsdrahtes eine Anschlußbuchse für eine Ableitung zu einem EKG-Gerät vorgesehen sein.

Als besonders überraschend an dieser Lösung muß gelten, daß mit der erfindungsgemäßen Vorrichtung ein einwandfreies EKG abgeleitet werden kann, obwohl die Elektrodenfenster durch den Katheterschlauch abgedeckt sind und somit nur das in den schmalen Zwischenraum zwischen Führungsmandrin und Innenseite des Katheterschlauches eingedrungene Blut zur Übertragung der zu messenden Aktionspotentiale zur Verfügung steht.

In der EP-A1-0 082 504 ist ein Venenkatheter und die Vorgangsweise beim Einführen des Katheters beschrieben. In einer speziellen Ausführungsform dieses Venenkatheters ist als Führungsmandrin ein kunststoffummantelter Metalldraht vorgesehen, wobei der Metalldraht bereits vor Beginn des halbkreisförmig gekrümmten distalen Endes des Führungsmandrins endet. Dieser Venenkatheter nach der EP-A1-0 082 504 kann durch einfache Modifikation in einen erfindungsgemäßen Venenkatheter verwandelt werden: Der Metalldraht samt Kunststoffummantelung wird nach proximal verlängert und mit einer Anschlußbuchse für eine Ableitung zu einem EKG-Gerät versehen. In der Nähe des distalen Endes des Führungsdrahts, jedoch vor Beginn des halbkreisförmigen gebogenen Stückes, wird die Kunststoffummantelung zur Schaffung von Elektrodenfenstern an einer oder mehreren Stellen, die innerhalb des Katheterschlauchs liegen ausgespart.

Entsprechendes gilt für den in der DE-PS 31 00 545 beschriebenen Venenkatheter. Durch Ersatz des dort vorgesehenen Führungsmandrins mit gebogenem distalen Ende durch einen Führungsmandrin mit den kennzeichnenden Merkmalen des Patentanspruchs der vorliegenden Anmeldung erhält man einen Venenkatheter mit der Möglichkeit der Lagekontrolle durch EKG.

Das Einführen derartiger Venenkatheter in eine Vene mit Hilfe der üblichen Punktionsbestecke und Punktionstechniken ist dem Fachmann geläufig und auch in der EP-A1-0 082 504 und der DE-PS 31 00 545 beschrieben. Nach Einführung in eine Vene wird der Katheter mit dem Führungsmandrin unter EKG-Kontrolle, wobei das halbkreisförmig gebogene distale Ende des Führungsmandrins aus dem distalen Ende des Katheterschlauchs herausragt, vorgeschoben, bis die charakteristischen Signale eines intracardialen EKG beobachtet werden können. Sodann wird der Katheter samt Führungsmandrin zurückgezogen, bis ein «normales», einer extracardialen Lage des distalen Katheterendes entsprechendes EKG beobachtet werden kann. Der Führungsmandrin kann nunmehr nach proximal aus dem Katheter herausgezogen werden und der Katheter mit der gewünschten Einrichtung, beispielsweise einem Gerät zur Bestimmung des zentralvenösen Druckes und/oder einer zu einer Infusionsflasche führenden Leitung verbunden werden.

Die Dimensionierung eines erfindungsgemäßen Venenkatheters entspricht der für bei Venenkatheterungen üblichen. Angaben zur Dimensionierung finden sich beispielsweise in der zitierten EP-A1-0 082 504. Der dort beschriebene Venenkatheter ist insbesondere für zentralvenöse Katheterungen über periphere Zugänge, worunter Zugänge über Arm- und Beinvenen verstanden werden, ausgelegt.

Die Durchbrechungen der Kunststoffummantelung des Führungsmandrins, im Zusammenhang mit dieser Erfindung als Elektrodenfenster bezeichnet, sind bezüglich Anzahl, äußerer Form und Lage auf dem Führungsmandrin in weiten Grenzen variierbar. Diese Elektrodenfenster können dabei beispielsweise paarig gegenüberliegend angeordnet sein, wobei benachbarte Paare gegeneinander versetzt, beispielsweise kreuzweise versetzt sein können. Wichtig ist, daß die Elektrodenfenster so angebracht sind, daß sie beim Vorschieben des Katheters vom Katheterschlauch bedeckt sind. Zweckmäßigerweise sind sie möglichst in der Nähe des distalen gekrümmten Endes des Führungsmandrins angebracht. Es hat sich jedoch gezeigt, daß die Elektrodenfenster mindestens bis etwa ein Drittel der Katheterlänge vom distalen Ende entfernt angebracht sein können, ohne daß störende Qualitätsminderungen des EKG auftreten. Daher ist es auch möglich, den Führungsmandrin so zu gestalten, daß dessen Metalldraht bereits eine gewisse Strecke vor dem gekrümmten distalen Ende endet, beispielsweise 1 bis 6 cm davor. Vorzugsweise weist das den Metalldraht überragende Ende eine Länge von 1,5 bis 2,5 cm auf.

In einer bevorzugten Ausführungsform endet der Metalldraht etwa 15 mm vor dem distalen Ende des Führungsmandrins. Das überstehende Teil der Kunststoffummantelung ist halbkreisförmig gebogen. Ein erstes Paar von sich gegenüberliegenden Elektrodenfenstern befindet sich in einem Abstand von 2 mm nach proximal vom distalen Ende des Metalldrahts. Ein weiteres Paar von Elektrodenfenstern ist in einem Abstand von 7 mm in proximaler Richtung vom distalen Ende des Metalldrahts vorgesehen. Die Richtungen der Achsen der beiden Elektrodenfensterpaare stehen aufeinander senkrecht.

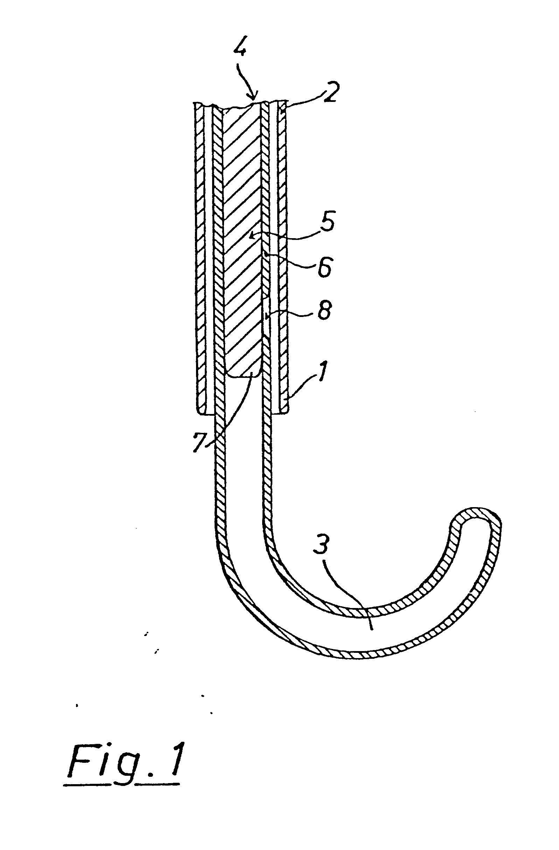

Nachstehend wird die Erfindung anhand von Fig. 1 näher erläutert.

Fig. 1 zeigt im Querschnitt das distale Ende eines Venenkatheters. Aus dem distalen Ende 1 eines Katheterschlauchs 2 ragt das in etwa halbkreisförmig gekrümmte distale Ende 3 des Führungsmandrins 4 hervor. Der Führungsmandrin 4 besteht aus dem Metalldraht 5 und der Kunststoffummantelung 6, welche über das distale Ende 7 des Metalldrahts 5 hinausragt und das halbkreisförmig gekrümmte distale Ende 3 des Führungsmandrins 4 bildet. In der Nähe des distalen Endes 7 des Metalldrahts 5 weist die Kunststoffummantelung 6 eine Aussparung 8 auf, die als Elektrodenfenster wirkt.

## Patentansprüche

1. Venenkatheter mit einem Katheterschlauch (2) und einem kunststoffummantelten, elektrisch leitfähigen Metalldraht (5) als Führungsmandrin, über den zum Zwecke der Lagekontrolle ein EKG abgeleitet werden kann, dadurch gekennzeichnet, daß die Kunststoffummantelung (6) im Bereich des distalen Endes (7) des Metalldrahts (5) eine oder mehrere Aussparungen (8) aufweist, die sich innerhalb des Lumens des Katheterschlauches (2) befinden.

2. Venenkatheter nach Anspruch 1, dadurch gekennzeichnet, daß sich die Aussparungen (8) in einer Entfernung von 1 bis 30 mm vom distalen Ende (7) des Metalldrahts (5) befinden.

3. Venenkatheter nach Anspruch 1 oder 2, dadurch gekennzeichnet, daß die Kunststoffummantelung (6) über das distale Ende (7) des Metalldrahts (5) in einer Länge von 1 bis 6 cm hinausragt.

4. Venenkatheter nach Anspruch 3, dadurch gekennzeichnet, daß das das distale Ende (7) des Metalldrahts (5) überragende Teil der Kunststoffummantelung (6) in seiner ganzen Länge oder teilweise als halbkreisförmig gekrümmtes Ende (3) ausgeführt ist.

5. Venenkatheter nach Anspruch 1, dadurch gekennzeichnet, daß am proximalen Ende des Führungsdrahts (4) eine Anschlußbuchse für eine Ableitung zu einem EKG-Gerät vorgesehen ist.

## Claims

1. Vein catheter with a catheter tube (2) and a plastic-covered, electrically conductive metal wire (5) as guide, which, for the purpose of positional control, can be used as an ECG lead, characterized in that the plastic covering (6) has, in the area of the distal end (7) of the metal wire (5), one or more recesses (8) which are situated within the lumen of the catheter tube (2).

2. Vein catheter according to Claim 1, characterized in that the recesses (8) are situated at a distance of 1 to 30 mm from the distal end (7) of the metal wire (5).

3. Vein catheter according to Claim 1 or 2, characterized in that the plastic covering (6) projects beyond the distal end (7) of the metal wire (5) by a length of 1 to 6 cm.

4. Vein catheter according to Claim 3, characterized in that the part of the plastic covering (6) projecting beyond the distal end (7) of the metal wire (5) is designed, in its entire length or partially, as a semicircular curved end (3).

5. Vein catheter according to Claim 1, characterized in that, at the proximal end of the guide wire (4), a connection is provided for a lead to an ECG device.

**Revendications**

1. Cathéter pour le cathétérisme des veines, comportant un tube de cathéter (2) et un fil métallique (5) électriquement conducteur muni d'une gaine de matière plastique constituant un mandrin de guidage, propre à être branché à un ECG aux fins de contrôle de position, caractérisé en ce que la gaine de matière plastique (6) comporte au voisinage de l'extrémité distale (7) du fil métallique (5) un ou plusieurs évidements (8) qui se trouvent à l'intérieur de la lumière du tube de cathéter (2).

2. Cathéter selon la revendication 1, caractérisé en ce que les évidements (8) se trouvent à une distance de l'extrémité distale (7) du fil métallique (5) de 1 à 30 mm.

3. Cathéter selon la revendication 1 ou 2, caractérisé en ce que la gaine de matière plastique (6) dépasse de l'extrémité distale (7) du fil métallique (5) dur une longueur de 1 à 6 cm.

4. Cathéter selon la revendication 3, caractérisé en ce que la partie de la gaine de matière plastique (6) dépassant de l'extrémité distale (7) du fil métallique (5) est réalisée, sur toute sa longueur ou en partie, sous forme d'une extrémité (3) incurvée en demi-cercle.

5. Cathéter selon la revendication 1, caractérisé en ce qu'il est prévu à l'extrémité distale du fil de guidage (4) une douille de connexion pour un branchement sur un appareil d'électrocardiographie.

_Fig.1_